# EUROPÄISCHE PATENTSCHRIFT

(11) **EP 1 373 214 B1**
(45) Veröffentlichungstag und Bekanntmachung des Hinweises auf die Patenterteilung: **22.08.2007**
(21) Anmeldenummer: 02716770.9
(22) Anmeldetag: 14.02.2002
(51) Int. Cl.: C07D 221/18, C07D 491/06, C09B 3/14, C09B 3/18, C09B 3/20

(54) **RYLENDERIVATE UND DEREN VERWENDUNG ALS FARBSTOFFE**
RYLENE DERIVATIVES AND THEIR USE AS DYES
DERIVES RYLENES ET LEUR UTILISATION COMME COLORANTS

(30) Priorität: 20.02.2001 DE 10108156
(43) Veröffentlichungstag der Anmeldung: 02.01.2004
(73) Patentinhaber: BASF Aktiengesellschaft, 67056 Ludwigshafen (DE); Max-Planck-Gesellschaft zur Förderung der Wissenschaften e.V., 80539 München (DE)
(72) Erfinder: KRIEGER, Matthias, 68167 Mannheim (DE); BÖHM, Arno, 68305 Mannheim (DE); REUTHER, Erik, 55122 Mainz (DE); MÜLLEN, Klaus, 50939 Köln (DE)
(86) Internationale Anmeldenummer: PCT/EP2002/001556
(87) Internationale Veröffentlichungsnummer: WO 2002/066438

(56) Entgegenhaltungen:
- DE-A- 4 338 784
- DE-A- 19 512 773
- US-A- 4 599 408
- LANGHALS H ET AL: "A Two-Step Synthesis of Quaterrylenetetracarboxylic Bisimides-Novel NIR Fluorescent Dyes" TETRAHEDRON LETTERS, ELSEVIER SCIENCE PUBLISHERS, AMSTERDAM, NL, Bd. 36, Nr. 36, 4. September 1995 (1995-09-04), Seiten 6423-6424, XP004027248 ISSN: 0040-4039

## Beschreibung

Die vorliegende Erfindung betrifft neue Rylenderivate der allgemeinen Formel I 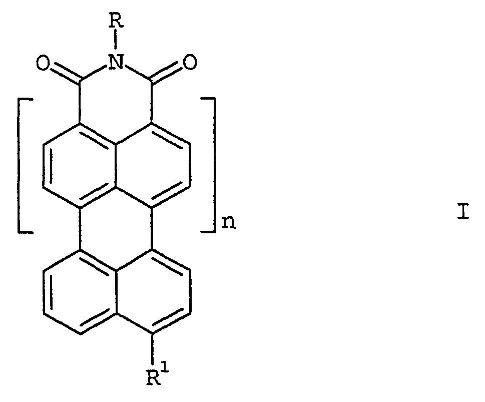 in der die Variablen folgende Bedeutung haben:
- R: Wasserstoff;
C₁-C₃₀-Alkyl, dessen Kohlenstoffkette durch eine oder mehrere Gruppierungen -O-, -S-, -NR²-, -CO- und/oder -SO₂- unter-brochen sein kann und das durch Carboxy, Sulfo, Hydroxy, Cyano, C₁-C₆-Alkoxy und/oder einen über ein Stickstoffatom gebundenen, 5- bis 7-gliedrigen heterocyclischen Rest, der weitere Heteroatome enthalten und aromatisch sein kann, ein-oder mehrfach substituiert sein kann;
Aryl oder Hetaryl, das durch C₁-C₁₈-Alkyl, C₁-C₆-Alkoxy, Halogen, Hydroxy, Cyano, Carboxy, -CONHR³, -NHCOR³ und/oder Aryl-oder Hetarylazo, das jeweils durch C₁-C₁₀-Alkyl, C₁-C₆-Alkoxy, Halogen, Hydroxy, Cyano und/oder Carboxy substituiert sein kann, ein- oder mehrfach substituiert sein kann;
- R¹: Wasserstoff oder Brom;
- R²: Wasserstoff oder C₁-C₆-Alkyl;
- R³: Wasserstoff; C₁-C₁₈-Alkyl ; Aryl oder Hetaryl, das jeweils durch C₁-C₈-Alkyl, C₁-C₆-Alkoxy, Halogen, Hydroxy und/oder Cyano substituiert sein kann;
- n: 2 oder 3,
sowie die Herstellung der Rylenderivate I und ihre Verwendung zur Einfärbung von hochmolekularen organischen und anorganischen Materialien, insbesondere von Kunststoffen, Lacken und Druckfarben, als Dispergierhilfsmittel, Pigmentadditive für organische Pigmente und Zwischenprodukte für die Herstellung von Fluoreszenzfarbmitteln und Pigmentadditiven, als farbgebende Komponente in der dekorativen Kosmetik, zur Herstellung farbiger oder im nahinfraroten Bereich des elektromagnetischen Spektrums absorbierender und/oder emittierender wäßriger Polymerisatdispersionen, als Photoleiter in der Elektrophotographie, als Emitter in Elektro- und Chemilumineszenzanwendungen, als Aktivkomponenten in der Fluoreszenzkonversion und als Laserfarbstoffe.

Außerdem betrifft die Erfindung neue Rylentetracarbonsäuremonoimidmonoanhydride der allgemeinen Formel III 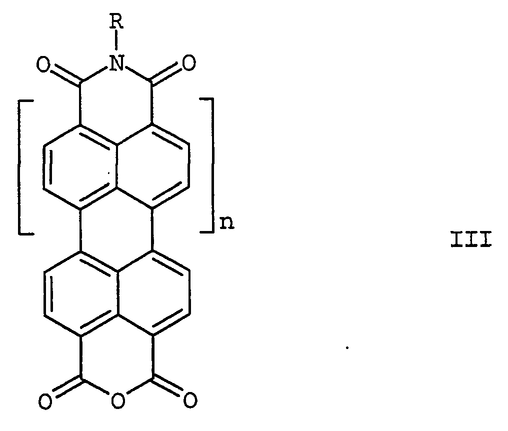 in der die Variablen die oben angegebene Bedeutung haben, als Zwischenprodukte für die Darstellung der Rylenderivate I sowie ihre Herstellung und Verwendung zur Einfärbung von hochmolekularen organischen und anorganischen Materialien, als Dispergierhilfsmittel, Pigmentadditive für organische Pigmente und Zwischenprodukte für die Herstellung polymerisierbarer Farbmittel und Pigmentadditive, als farbgebende Komponente in der dekorativen Kosmetik, zur Herstellung farbiger oder im nahinfraroten Bereich des elektromagnetischen Spektrums absorbierender und/oder emittierender wäßriger Polymerisatdispersionen, als Photoleiter in der Elektrophotographie, als Emitter in Elektro- und Chemilumineszenzanwendungen, als Aktivkomponenten in der Fluoreszenzkonversion und als Laserfarbstoffe.

Kürzere Rylen-3,4,-dicarbonsäureimide (mit n = 0 oder 1 in Formel I, d.h. entsprechende Naphthalin-und Perylenderivate) sind bekannt. Unsubstituierte bzw. in der peri-Position (9-Position des Perylengerüsts) durch Halogen substituierte Perylen-3,4-dicarbon-säureimide werden beispielsweise in der EP-A-636 666, in Chimia 48, S. 503 - 505 (1994) sowie in der EP-A-592 292 und in Dyes and Pigments 16, S. 19 - 25 (1991) beschrieben. Weitere zusätzlich im Perylengerüst subsitituierte 9-Halogenperylen-3,4-dicarbonsäure-imide sind in der WO-A-96/22332 offenbart. DE 4338784 beschreibt die Herstellung von Perylen-3,4 dicarbonsäureimiden. Den bekannten Verbindungen ist gemeinsam, daß sie im nahen UV oder dem kurzwelligen Bereich des sichtbaren Spektrums, d.h. im wesentlichen im Bereich von 340 bis 540 nm, absorbieren.

Der Erfindung lag die Aufgabe zugrunde, Rylenderivate bereitzustellen, die im langwelligen, d.h. im roten und infraroten, Bereich des elektromagnetischen Spektrums absorbieren und emittieren und in der peri-Position gezielt derivatisierbar sind.

Demgemäß wurden die Terrylen- und Quaterrylenderivate der eingangs definierten Formel I gefunden.

Bevorzugte Rylenderivate sind dem Unteranspruch zu entnehmen.

Weiterhin wurde ein Verfahren zur Herstellung der Rylenderivate I gefunden, welches dadurch gekennzeichnet ist, daß man
a) ein asymmetrisches Rylentetracarbonsäurediimid der allgemeinen Formel II 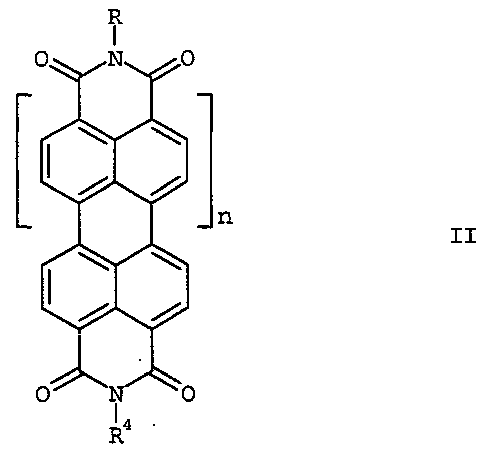 in der R⁴ C₅-C₈-Cycloalkyl, dessen Kohlenstoffgerüst durch eine oder mehrere Gruppierungen -O-, -S- und/oder -NR²- unterbrochen sein kann und das durch C₁-C₆-Alkyl ein- oder mehrfach substituiert sein kann, bedeutet,
   unter alkalischen Bedingungen in Gegenwart eines polaren organischen Lösungsmittels einseitig verseift,
b) das in Schritt a) gebildete Rylentetracarbonsäuremonoimidmonoanhydrid der allgemeinen Formel III 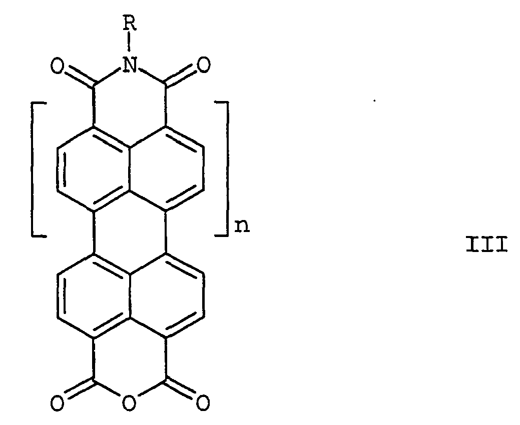 in Gegenwart einer tertiären stickstoffbasischen Verbindung und eines Übergangsmetallkatalysators einseitig decarboxyliert und
c) das Reaktionsprodukt gewünschtenfalls abschließend mit elementarem Brom umsetzt.

Außerdem wurde ein Verfahren zur Herstellung der Rylentetracarbonsäuremonoimidmonoanhydride III gefunden, welches dadurch gekennzeichnet ist, daß man ein asymmetrisches Rylentetracarbonsäurediimid II unter alkalischen Bedingungen in Gegenwart eines polaren organischen Lösungsmittels einseitig verseift.

Zudem wurden die Rylentetracarbonsäuremonoimidmonoanhydride der eingangs definierten Formel III als Zwischenprodukte für die Rylen-3,4-dicarbonsäureimide I gefunden.

Nicht zuletzt wurden die ebenfalls eingangs genannten Anwendungszwecke für die Rylenderivate I und die Rylentetracarbonsäuremonoimidmonoanhydride III gefunden.

Alle in den Formeln I bis III auftretenden Alkylgruppen können geradkettig oder verzweigt sein. Wenn die Alkylgruppen substituiert sind, tragen sie in der Regel 1 oder 2 Substituenten.

Als Beispiele für geeignete Reste R, R¹, R², R³ und R⁴ (bzw. für deren Substituenten) seien im einzelnen genannt:
Methyl, Ethyl, Propyl, Isopropyl, Butyl, Isobutyl, tert.-Butyl, Pentyl, Isopentyl, Neopentyl, tert.-Pentyl, Hexyl, 2-Methylpentyl, Heptyl, 1-Ethylpentyl, Octyl, 2-Ethylhexyl, Isooctyl, Nonyl, Isononyl, Decyl, Isodecyl, Undecyl, Dodecyl, Tridecyl, Isotridecyl, Tetradecyl, Pentadecyl, Hexadecyl, Heptadecyl, Octadecyl, Nonadecyl und Eicosyl (die obigen Bezeichnungen Isooctyl, Isononyl, Isodecyl und Isotridecyl sind Trivialbezeichnungen und stammen von den nach der Oxosynthese erhaltenen Alkoholen);
2-Methoxyethyl, 2-Ethoxyethyl, 2-Propoxyethyl, 2-Isopropoxyethyl, 2-Butoxyethyl, 2- und 3-Methoxypropyl, 2- und 3-Ethoxypropyl, 2-und 3-Propoxypropyl, 2- und 3-Butoxypropyl, 2- und 4-Methoxybutyl, 2- und 4-Ethoxybutyl, 2- und 4-Propoxybutyl, 3,6-Dioxaheptyl, 3,6-Dioxaoctyl, 4,8-Dioxanonyl, 3,7-Dioxaoctyl, 3,7-Dioxanonyl, 4,7-Dioxaoctyl, 4,7-Dioxanonyl, 2- und 4-Butoxybutyl, 4,8-Dioxadecyl, 3,6,9-Trioxadecyl, 3,6,9-Trioxaundecyl, 3,6,9-Trioxadodecyl, 3,6,9,12-Tetraoxatridecyl und 3,6,9,12-Tetraoxatetradecyl;
2-Methylthioethyl, 2-Ethylthioethyl, 2-Propylthioethyl, 2-Isopropylthioethyl, 2-Butylthioethyl, 2- und 3-Methylthiopropyl, 2-und 3-Ethylthiopropyl, 2- und 3-Propylthiopropyl, 2- und 3-Butylthiopropyl, 2- und 4-Methylthiobutyl, 2- und 4-Ethylthiobutyl, 2-und 4-Propylthiobutyl, 3,6-Dithiaheptyl, 3,6-Dithiaoctyl, 4,8-Dithianonyl, 3,7-Dithiaoctyl, 3,7-Dithianonyl, 2- und 4-Butylthiobutyl, 4,8-Dithiadecyl, 3,6,9-Trithiadecyl, 3,6,9-Trithiaundecyl, 3,6,9-Trithiadodecyl, 3,6,9,12-Tetrathiatridecyl und 3,6,9,12-Tetrathiatetradecyl;
2-Monomethyl- und 2-Monoethylaminoethyl, 2-Dimethylaminoethyl, 2-und 3- Dimethylaminopropyl, 3-Monoisopropylaminopropyl, 2- und 4-Monopropylaminobutyl, 2- und 4-Dimethylaminobutyl, 6-Methyl-3,6-diazaheptyl, 3,6-Dimethyl-3,6-diazaheptyl, 3,6-Diazaoctyl, 3,6-Dimethyl-3,6-diazaoctyl, 9-Methyl-3,6,9-triazadecyl, 3,6,9-Trimethyl-3,6,9-triazadecyl, 3,6,9-Triazaundecyl, 3,6,9-Trimethyl-3,6,9-triazaundecyl, 12-Methyl-3,6,9,12-tetraazatridecyl und 3,6,9,12-Tetramethyl-3,6,9,12-tetraazatridecyl;
Propan-2-on-1-yl, Butan-3-on-1-yl, Butan-3-on-2-yl und 2-Ethyl-pentan-3-on-1-yl;
2-Methylsulfonylethyl, 2-Ethylsulfonylethyl, 2-Propylsulfonylethyl, 2-Isopropylsulfonylethyl, 2-Butylsulfonylethyl, 2- und 3-Methylsulfonylpropyl, 2- und 3-Ethylsulfonylpropyl, 2- und 3-Propylsulfonylpropyl, 2- und 3-Butylsulfonylpropyl, 2- und 4-Methylsulfonylbutyl, 2- und 4-Ethylsulfonylbutyl, 2- und 4-Propylsulfonylbutyl und 4-Butylsulfonylbutyl;
Carboxymethyl, 2-Carboxyethyl, 3-Carboxypropyl, 4-Carboxybutyl, 5-Carboxypentyl, 6-Carboxyhexyl, 8-Carboxyoctyl, 10-Carboxydecyl, 12-Carboxydodecyl und 14-Carboxytetradecyl;
Sulfomethyl, 2-Sulfoethyl, 3-Sulfopropyl, 4-Sulfobutyl, 5-Sulfopentyl, 6-Sulfohexyl, 8-Sulfooctyl, 10-Sulfodecyl, 12-Sulfododecyl und 14-Sulfotetradecyl;
2-Hydroxethyl, 3-Hydroxypropyl, 1-Hydroxyprop-2-yl, 2- und 4-Hydroxybutyl, 1-Hydroxybut-2-yl und 8-Hydroxy-4-oxaoctyl;
Cyanomethyl, 2-Cyanoethyl, 3-Cyanopropyl, 2-Methyl-3-ethyl-3-cyanopropyl, 7-Cyano-7-ethylheptyl und 4,7-Dimethyl-7-cyanoheptyl;
Methoxy, Ethoxy, Propoxy, Isopropoxy, Butoxy, Isobutoxy, sec.-Butoxy, tert.-Butoxy, Pentoxy, Isopentoxy, Neopentoxy, tert.-Pentoxy und Hexoxy;
Carbamoyl, Methylaminocarbonyl, Ethylaminocarbonyl, Propylaminocarbonyl, Butylaminocarbonyl, Pentylaminocarbonyl, Hexylaminocarbonyl, Heptylaminocarbonyl, Octylaminocarbonyl, Nonylaminocarbonyl, Decylaminocarbonyl und Phenylaminocarbonyl;
Formylamino, Acetylamino, Propionylamino und Benzoylamino;
Chlor, Brom und Iod;
Phenylazo, 2-Napthylazo, 2-Pyridylazo und 2-Pyrimidylazo;
Phenyl, 2-Naphthyl, 2- und 3-Pyrryl, 2-, 3- und 4-Pyridyl, 2-, 4-und 5-Pyrimidyl, 3-, 4- und 5-Pyrazolyl, 2-, 4- und 5-Imidazolyl, 2-, 4- und 5-Thiazolyl, 3-(1,2,4-Triazyl), 2-(1,3,5-Triazyl), 6-Chinaldyl, 3-, 5-, 6- und 8-Chinolinyl, 2-Benzoxazolyl, 2-Benzothiazolyl, 5-Benzothiadiazolyl, 2- und 5-Benzimidazolyl und 1-und 5- Isochinolyl;
2-, 3- und 4-Methylphenyl, 2,4-, 2,5-, 3,5- und 2,6-Dimethylphenyl, 2,4,6-Trimethylphenyl, 2-, 3- und 4-Ethylphenyl, 2,4-, 2,5-, 3,5- und 2,6-Diethylphenyl, 2,4,6-Triethylphenyl, 2-, 3-und 4-Propylphenyl, 2,4-, 2,5-, 3,5- und 2,6-Dipropylphenyl, 2,4,6-Tripropylphenyl, 2-, 3- und 4-Isopropylphenyl, 2,4-, 2,5-, 3,5- und 2,6-Diisopropylphenyl, 2,4,6-Triisopropylphenyl, 2-, 3-und 4-Butylphenyl, 2,4-, 2,5-, 3,5- und 2,6-Dibutylphenyl, 2,4,6-Tributylphenyl, 2-, 3- und 4-Isobutylphenyl, 2,4-, 2,5-, 3,5- und 2,6-Diisobutylphenyl, 2,4,6-Triisobutylphenyl, 2-, 3-und 4-sec.-Butylphenyl, 2,4-, 2,5-, 3,5- und 2,6-Di-sec.-butylphenyl und 2,4,6-Tri-sec.-butylphenyl, 2-, 3- und 4-tert.-Butylphenyl, 2,4-, 2,5-, 3,5- und 2,6-Di-tert.-butylphenyl, 2,4,6-Tri-tert.-butylphenyl; 2-, 3- und 4-Methoxyphenyl, 2,4-, 2,5-, 3,5-und 2,6-Dimethoxyphenyl, 2,4,6-Trimethoxyphenyl, 2-, 3- und 4-Ethoxyphenyl, 2,4-, 2,5-, 3,5- und 2,6-Diethoxyphenyl, 2,4,6-Triethoxyphenyl, 2-, 3- und 4-Propoxyphenyl, 2,4-, 2,5-, 3,5- und 2,6-Dipropoxyphenyl, 2-, 3- und 4-Isopropoxyphenyl, 2,4-, 2,5-, 3,5- und 2,6-Diisopropoxyphenyl und 2-, 3- und 4-Butoxyphenyl; 2-, 3- und 4-Chlorphenyl, und 2,4-, 2,5-, 3,5-und 2,6-Dichlorphenyl; 2-, 3- und 4-Hydroxyphenyl und 2,4-, 2,5-, 3,5- und 2,6-Dihydroxyphenyl; 2-, 3- und 4-Cyanophenyl; 3- und 4-Carboxyphenyl; 3- und 4-Carboxamidophenyl, 3- und 4-N-Methylcarboxamidophenyl und 3- und 4-N-Ethylcarboxamidophenyl; 3- und 4-Acetylaminophenyl, 3- und 4-Propionylaminophenyl und 3- und 4-Butyrylaminophenyl; 3- und 4-N-Phenylaminophenyl, 3- und 4-N-(o-Tolyl)aminophenyl, 3- und 4-N-(m-Tolyl)aminophenyl und 3-und 4-N-(p-Tolyl)aminophenyl; 3- und 4-(2-Pyridyl)aminophenyl, 3-und 4-(3-Pyridyl)aminophenyl, 3- und 4-(4-Pyridyl)aminophenyl, 3-und 4-(2-Pyrimidyl)aminophenyl und 4-(4-Pyrimidyl)aminophenyl;
4-Phenylazophenyl, 4-(1-Naphthylazo)phenyl, 4-(2-Naphthylazo)phenyl, 4-(4-Naphthylazo)phenyl, 4-(2-Pyridylazo)phenyl, 4-(3-Pyridylazo)phenyl, 4-(4-Pyridylazo)phenyl, 4-(2-Pyrimidylazo)phenyl, 4-(4-Pyrimidylazo)phenyl und 4-(5-Pyrimidylazo) phenyl;
Cyclopentyl, 2- und 3-Methylcyclopentyl, 2- und 3-Ethylcyclopentyl, Cyclohexyl, 2-, 3- und 4-Methylcyclohexyl, 2-, 3- und 4-Ethylcyclohexyl, 3- und 4-Propylcyclohexyl, 3- und 4-Isopropylcyclohexyl, 3- und 4-Butylcyclohexyl, 3- und 4-sec.-Butylcyclohexyl, 3- und 4-tert.-Butylcyclohexyl, Cycloheptyl, 2-, 3- und 4-Methylcycloheptyl, 2-, 3- und 4-Ethylcycloheptyl, 3- und 4-Propylcycloheptyl, 3- und 4-Isopropylcycloheptyl, 3- und 4-Butylcycloheptyl, 3- und 4-sec.-Butylcycloheptyl, 3- und 4-tert.-Butylcycloheptyl, Cyclooctyl, 2-,3-, 4- und 5-Methylcyclooctyl, 2-, 3-, 4- und 5-Ethylcyclooctyl, 3-, 4- und 5-Propylcyclooctyl, 2-Dioxanyl, 4-Morpholinyl, 2- und 3-Tetrahydrofuryl, 1-, 2- und 3-Pyrrolidinyl und 1-, 2-, 3- und 4-Piperidyl.

Die Herstellung der Rylenderivate I kann vorteilhaft nach dem erfindungsgemäßen mehrstufigen Verfahren erfolgen, bei dem in Schritt a) ein asymmetrisches, an einem Imidstickstoff durch einen Cycloalkylrest substituiertes Rylentetracarbonsäurediimid II einseitig verseift wird und in Schritt b) das gebildete Rylentetracarbonsäuremonoimidmonoanhydrid III einseitig decarboxyliert wird. Ist ein in peri-Position bromiertes Rylenderivat I (R¹ = Br) gewünscht, so wird ein in der peri-Position unsubstituiertes Rylenderivat I (R¹ = H) noch in einem weiteren Schritt c) mit elementarem Brom umgesetzt.

Schritt a) des erfindungsgemäßen Verfahrens, die Verseifung der cycloalkylsubstituierten Imidgruppe der Rylentetracarbonsäurediimide II, wird in Gegenwart eines polaren organischen Lösungsmittels und einer Base vorgenommen.

Die hierfür als Edukte eingesetzten asymmetrischen Rylentetracarbonsäurediimide II sind bekannt und können wie in der EP-A-592 292 und in Chem. Eur. Journal 3, S. 219 - 225 (1997) beschrieben hergestellt werden.

Geeignete Lösungsmittel sind insbesondere verzweigte C₃-C₆-Alkohole, wie Isopropanol, tert.-Butanol und 2-Methyl-2-butanol.

Im allgemeinen kommen 40 bis 200 g Lösungsmittel je g II zum Einsatz.

Als Basen eignen sich anorganische Basen, insbesondere Alkali-und Erdalkalimetallhydroxide, z.B. Natriumhydroxid und Kaliumhydroxid, die vorzugsweise in Form von wäßrigen Lösungen bzw. Suspensionen (in der Regel 50 bis 80 gew.-%ig) verwendet werden, und organische Basen, insbesondere Alkali- und Erdalkalimetallalkoxide, wobei Natrium- und Kaliumalkoxide, wie Natriummethylat, Kaliummethylat, Kaliumisopropylat und Kalium-tert.-butylat bevorzugt sind, die üblicherweise in wasserfreier Form eingesetzt werden.

In der Regel werden 5 bis 50 Äquivalente Base, bezogen auf II, benötigt.

Die Reaktionstemperatur beträgt im allgemeinen 50 bis 120°C, vorzugsweise 60 bis 100°C.

Die Verseifung ist üblicherweise in 10 bis 40 h abgeschlossen.

Verfahrenstechnisch geht man in Schritt a) zweckmäßigerweise wie folgt vor:

Man erhitzt eine Suspension aus Rylentetracarbonsäurediimid II, Base und Lösungsmittel unter starkem Rühren auf die gewünschte Reaktionstemperatur. Nach beendeter Verseifung und Abkühlen auf Raumtemperatur stellt man mit einer Säure, z.B. einer anorganischen Säure, wie Salzsäure, oder einer organischen Säuren, wie Essigsäure, einen pH-Wert von 1 bis 4 ein. Man filtriert das Produkt ab, wäscht mit heißem Wasser und trocknet anschließend im Vakuum bei etwa 100°C.

In der Regel hat das in Schritt a) erhaltene Rylentetracarbonsäuremonoimidmonoanhydrid III bereits einen so hohen Wertgehalt (> 90%), daß es direkt für die Decarboxylierung in Schritt b) eingesetzt werden kann.

In Schritt b) des erfindungsgemäßen Verfahrens werden die Rylentetracarbonsäuremonoimidmonoanhydride III in Gegenwart einer tertiären stickstoffbasischen Verbindung als Lösungsmittel und eines Übergangsmetallkatalysators einseitig decarboxyliert.

Geeignete Lösungsmittel sind insbesondere hochsiedende Stickstoffbasen, z.B. cyclische Amide, wie N-Methylpyrrolidon, und aromatische Heterocyclen, wie Chinolin, Isochinolin und Chinaldin.

Übliche Lösungsmittelmengen betragen 20 bis 150 g je g III.

Als Katalysatoren eignen sich insbesondere die Übergangsmetalle Kupfer und Zink sowie besonders auch deren anorganische und organische Salze, die vorzugsweise in wasserfreier Form eingesetzt werden.

Beispiele für bevorzugte Salze sind Kupfer(I)oxid, Kupfer(II)oxid, Kupfer(I)chlorid, Kupfer(II)acetat, Zinkacetat und Zinkpropionat, wobei Kupfer(I)oxid und Zinkacetat besonders bevorzugt sind.

Selbstverständlich kann man auch Mischungen der genannten Katalysatoren verwenden.

In der Regel kommen 50 bis 90 mol-% Katalysator, bezogen auf III, zum Einsatz.

Die Reaktionstemperatur liegt im allgemeinen bei 100 bis 250°C, insbesondere bei 160 bis 200°C. Es empfiehlt sich, unter Verwendung einer Schutzgasatmosphäre (z.B. Stickstoff) zu arbeiten.

Üblicherweise ist die Decarboxylierung in 3 bis 20 h beendet.

Verfahrenstechnisch geht man in Schritt b) zweckmäßigerweise wie folgt vor:

Man erhitzt eine Mischung aus Rylentetracarbonsäuremonoimidmonoanhydrid III, Lösungsmittel und Katalysator unter Rühren auf die gewünschte Reaktionstemperatur. Nach beendeter Decarboxylierung und Abkühlen auf Raumtemperatur gießt man das Reaktionsgemisch auf etwa das gleiche Volumen eines niedrig siedenden Alkohols, z.B. Methanol, versetzt unter Rühren mit der gleichen Menge einer verdünnten anorganischen Säure, z.B. einer 5-6 gew.%-igen Salzsäure und filtriert das Produkt ab. Gewünschtenfalls rührt man das Rohprodukt in einem geeigneten Lösungsmittel, z.B. einem aromatischen Lösungsmittel wie Xylol, auf, filtriert, wäscht mit einem niedrig siedenden Alkohol und trocknet es im Vakuum bei etwa 75°C.

Eine weitere Aufreinigung ist durch Umkristallisation aus einem hochsiedenden Lösungsmittel, z.B. N-Methylpyrrolidon, möglich. In der Regel ist dies jedoch nicht erforderlich, da die erhaltenen Rylenderivate I (R¹ = H) bereits einen Wertgehalt von ≥ 95% aufweisen.

Zur Herstellung der in peri-Position bromierten Rylenderivate I (R¹ = Br) können die unsubstituierten Rylenderivate I noch dem erfindungsgemäßen Schritt c), der regioselektiven Bromierung, unterzogen werden.

Diese Bromierung wird vorzugsweise in einer aliphatischen Monocarbonsäure, insbesondere einer C₁-C₄-Carbonsäure, wie Ameisensäure, Essigsäure, Propionsäure, Buttersäure oder deren Mischungen, oder in einem halogenierten, aliphatischen oder aromatischen Lösungsmittel, wie Methylenchlorid, Chloroform oder Chlorbenzol, durchgeführt.

Üblicherweise werden 5 bis 30 g, vorzugsgweise 15 bis 25 g, Lösungsmittel je g zu bromierendes I eingesetzt.

In der Regel ist die Anwesenheit eines Halogenierungskatalysators nicht erforderlich. Will man jedoch die Bromierungsreaktion beschleunigen (etwa um den Faktor 1,5 bis 2), so empfiehlt es sich, elementares Iod, bevorzugt in einer Menge von 1 bis 5 mol-%, bezogen auf I, zuzusetzen.

Im allgemeinen liegt das Molverhältnis von Brom zu I bei etwa 1 : 1 bis 5 : 1, vorzugsweise bei 3 : 1 bis 4 : 1.

Die Reaktionstemperatur beträgt in der Regel 0 bis 70°C, insbesondere 10 bis 40°C.

In Abhängigkeit von der Reaktivität des Substrats I und der Anoder Abwesenheit von Iod ist die Bromierung üblicherweise in 2 bis 12 h beendet.

Verfahrenstechnisch geht man in Schritt c) zweckmäßigerweise wie folgt vor:

Man stellt eine Mischung aus dem zu bromierenden Rylenderivat I und Lösungsmittel unter Rühren in 15 bis 30 min auf die gewünschte Reaktionstemperatur ein, setzt gegebenenfalls Katalysator und anschließend in 5 bis 10 min die gewünschte Brommenge zu und rührt die Mischung unter Lichtausschluß 2 bis 12 h bei der Reaktionstemperatur. Nach Entfernen von überschüssigem Brom mit einem kräftigen Stickstoffstrom trägt man das Reaktionsgemisch in die etwas gleiche Menge eines aliphatischen Alkohols, z.B. Methanol, ein, rührt über Nacht, filtriert das ausgefallene Produkt ab, wäscht es vorzugsweise mit dem gleichen Alkohol und trocknet es unter Vakuum bei etwa 120°C.

In der Regel haben auch die in Schritt c) erhaltenen bromierten Rylenderivate I bereits einen so hohen Wertgehalt (> 95%), daß sie direkt für den gewünschten Anwendungszweck eingesetzt werden können.

Die erfindungsgemäßen Rylenderivate I eignen sich hervorragend zur homogenen Einfärbung von hochmolekularen organischen und anorganischen Materialien, insbesondere z.B. von Kunststoffen, vor allem von thermoplastischen Kunststoffen, Lacken und Druckfarben sowie oxidischen Schichtsystemen.

Außerdem eignen sie sich als Dispergierhilfsmittel, Pigmentadditive für organische Pigmente und Zwischenprodukte für die Herstellung von Fluoreszenzfarbmitteln und Pigmentadditiven, als farbgebende Komponenten in der dekorativen Kosmetik, zur Herstellung farbiger oder im nahinfraroten Bereich des elektromagnetischen Spektrums absorbierender und/oder emittierender wäßriger Polymerisatdispersionen und als Photoleiter in der Elektrophotographie, als Emitter in Elektro- und Chemilumineszenzanwendungen, als Aktivkomponenten in der Fluoreszenzkonversion und als Laserfarbstoffe.

Auch die als Zwischenprodukte für die Rylenderivate I dienenden Rylentetracarbonsäuremonoimidmonoanhydride III können selbst zur Einfärbung der oben genannten hochmolekularen organischen und anorganischen Materialien, als Dispergierhilfsmittel, Pigmentadditive für organische Pigmente und Zwischenprodukte für die Herstellung polymerisierbarer Farbmittel und Pigmentadditive, als farbgebende Komponente in der dekorativen Kosmetik, zur Herstellung farbiger oder im nahinfraroten Bereich des elektromagnetischen Spektrums absorbierender und/oder emittierender wäßriger Polymerisatdispersionen, als Photoleiter in der Elektrophotographie, als Emitter in Elektro- und Chemilumineszenzanwendungen, als Aktivkomponenten in der Fluoreszenzkonversion und als Laserfarbstoffe eingesetzt werden.

### Beispiele

### A) Herstellung von erfindungsgemäßen Rylentetracarbonsäuremonoimidmonoanhydriden der Formel III

### Beispiele 1 bis 6

10 g (x mmol) Rylentetracarbonsäurediimid II wurden in 500 ml Isopropanol eingetragen und mit einem Gemisch aus 40 g Kaliumhydroxid und 30 ml Wasser versetzt. Die Mischung wurde dann t h unter Rühren zum Rückfluß erhitzt.

Nach Abkühlen auf Raumtemperatur wurde das Reaktionsgemisch in 300 ml eines Gemisches aus 2 Volumenteilen Wasser und einem Volumenteil konzentrierter Salzsäure gegebenen. Nach 30minütigem Rühren wurde das Produkt abfiltriert, mit Wasser bis zur pH-Neutralität gewaschen und im Vakuum bei 100°C getrocknet.

Weitere Einzelheiten zu diesen Versuchen sowie deren Ergebnisse sind in Tabelle 1 zusammengestellt.

**Tabelle 1**

| Bsp. | x [mmol] | Rylentetracarbonsäurediimid II | t [h] | Ausbeute [g]/[%] | Aussehen | Smp. [°C] |
|---|---|---|---|---|---|---|
| 1 | 13,2 | N-(2,6-Diisopropylphenyl)-N'-cyclohexylterrylen-3,4:11,12-tetracarbonsäurediimid | 20 | 8,9/100 | blauviolett, mikrokristallin | >350 |
| 2 | 14,2 | N-(p-Methoxyphenyl)-N'-cyclohexylterrylen-3,4:11,12-tetracarbonsäurediimid | 28 | 8,4/ 95 | schwarz-blau, amorph | >350 |
| 3 | 13,1 | N-Dodecyl-N'-cyclohexylterrylen-3,4:11,12-tetracarbonsäurediimid | 18 | 8,7/ 97 | schwarz, amorph | >350 |
| 4 | 11,4 | N-(2,6-Diisopropylphenyl)-N'-cyclohexylquaterrylen-3,4:13,14-tetracarbonsäurediimid | 15 | 8,9/ 98 | schwarz, mikrokristallin | >350 |
| 5 | 12,1 | N-(p-Methoxyphenyl)-N'-cyclohexylquaterrylen-3,4:13,14-tetracarbonsäurediimid | 20 | 8,3/ 92 | violett-schwarz, amorph | >350 |
| 6 | 11,3 | N-Dodecyl-N'-cyclohexylquaterrylen-3,4:13,14-tetracarbonsäurediimid | 28 | 8,6/ 95 | violett-schwarz, amorph | >350 |

### B) Herstellung von erfindungsgemäßen Rylenderivaten I (R¹ = H)

### Beispiele 7 bis 12

Eine Mischung aus x g (7 mmol) Rylentetracarbonsäuremonoimidmonoanhydrid III und 7 g Kupfer(I)oxid in 400 ml Chinolin wurde unter Rühren auf 220°C erhitzt und t h bei dieser Temperatur gehalten.

Nach Abkühlen auf Raumtemperatur wurde die Reaktionslösung in 400 ml Methanol eingetragen und mit 400 ml eines Gemisches aus 3 Volumenteilen Wasser und 1 Volumenteil konzentrierter Salzsäure versetzt. Nach 30minütigem Rühren wurde das Produkt abfiltriert, mit Wasser bis zur pH-Neutralität gewaschen und im Vakuum bei 75°C getrocknet. Anschließend wurde das so erhaltene Rohprodukt durch Umkristallisation aus N-Methylpyrrolidon von Spuren des Ausgangsmaterials befreit.

Weitere Einzelheiten zu diesen Versuchen sowie deren Ergebnisse sind in Tabelle 2 zusammengestellt.

**Tabelle 2**

| Bsp. | x [g] | Rylentetracarbonsäuremonoimidmonoanhydrid III | t [h] | Ausbeute [g]/[%] | Aussehen | Smp. [°C] |
|---|---|---|---|---|---|---|
| 7 | 4,8 | N-(2,6-Diisopropylphenyl)terrylen-3,4:11,12-tetracarbonsäuremonoimidmonoanhydrid | 10 | 3,7/88 | bläulichschwarz, mikrokristallin | >350 |
| 8 | 4,4 | N-(p-Methoxyphenyl)terrylen-3,4:11,12-tetracarbonsäuremonoimidmonoanhydrid | 12 | 3,3/85 | blau-schwarz, kristallin | >350 |
| 9 | 4,8 | N-Dodecylterrylen-3,4:11,12-tetracarbonsäuremonoimidmonoanhydrid | 15 | 3,9/90 | schwarz, amorph | >350 |
| 10 | 5,6 | N-(2,6-Diisopropylphenyl)quaterrylen-3,4:13,14-tetracarbonsäuremonoimidmonoanhydrid | 15 | 4,1/80 | schwarz, mikrokristallin | >350 |
| 11 | 5,2 | N-(p-Methoxyphenyl)quaterrylen-3,4:13,14-tetracarbonsäuremonoimidmonoanhydrid | 20 | 4,3/90 | schwarz, mikrokristallin | >350 |
| 12 | 5,7 | N-Dodecylquaterrylen-3,4:13,14-tetracarbonsäuremonoimidmonoanhydrid | 20 | 4,4/86 | schwarz, amorph | >350 |

| | | | | | | |
|---|---|---|---|---|---|---|
| Analytische Daten zu Beispiel 7: N-(2,6-Diisopropylphenyl)terrylen-3,4-dicarboximid: Elementaranalyse (Gew.-% ber./gef.): C: 87,25/87,4; H: 5,15/5,2; N: 2,3/2,2; O: 5,3/5,2; Masse (FD, 8kV): m/z = 605,2 (M⁺, 100 %); IR (KBr): ν = 1687 (s, C=O), 1647 (s, C=O) cm⁻¹; UV/VIS (NMP) : λₘₐₓ (ε) = 580 (42933), 639 (47376) nm. Analytische Daten zu Beispiel 10: N-(2,6-Diisopropylphenyl)quaterrylen-3,4-dicarboximid: Elementaranalyse (Gew.-% ber./gef.): C: 88,85/88,9; H: 4,85/4,8; N: 1,9/1,9; O: 4,4/4,4; Masse (FD, 8kV): m/z = 729,3 (M⁺, 100 %); IR (KBr): ν = 1690 (s, C=O), 1651 (s, C=O) cm⁻¹; UV/VIS (NMP) : λₘₐₓ (ε) = 677 (72355), 740 (80001) nm. | | | | | | |

### C) Herstellung von erfindungsgemäßen 11-Bromterrylen-3,4-dicarbonsäureimiden und 13-Bromquaterrylen-3,4-dicarbonsäure-imiden I (R¹ = Br)

### Beispiele 13 bis 18

x g (0,1 mol) Rylenderivat I wurden 30 min in 1,5 1 Eisessig suspendiert. Nach Zugabe von 1 g (3,9 mmol) Iod und 64 g (0,4 mol) Brom wurde die Mischung unter Lichtausschluß t h bei T°C gerührt.

Das Reaktionsgemisch wurde anschließend durch Hindurchleiten eines kräftigen Stickstoffstroms von überschüssigem Brom befreit, dann mit 1 1 Methanol verdünnt und über Nacht bei Raumtemperatur gerührt.

Das ausgefallene Produkt wurde abfiltriert, zunächst mit 1,5 1 Methanol und dann mit Wasser bis zum neutralen Ablauf gewaschen und bei 120°C im Vakuum getrocknet.

Weitere Einzelheiten zu diesen Versuchen sowie deren Ergebnisse sind in Tabelle 3 zusammengestellt.

**Tabelle 3**

| Bsp. | x [g] | Rylenderivat I | t [h] | T [°C] | Ausbeute [g]/[%] | Aussehen | Smp. [°C] |
|---|---|---|---|---|---|---|---|
| 13 | 60,6 | N-(2,6-Diisopropylphenyl)terrylen-3,4-dicarbonsäureimid | 8 | 30 | 61,6/90 | tiefblau, kristallin | >350 |
| 14 | 55,2 | N-(p-Methoxyphenyl)terrylen-3,4-dicarbonsäureimid | 12 | 40 | 56,7/90 | tiefblau, mikrokristallin | >350 |
| 15 | 61,4 | N-Dodecylterrylen-3,4-dicarbonsäureimid | 8 | 30 | 61,0/88 | tiefblau, mikrokristallin | >350 |
| 16 | 73,0 | N-(2,6-Diisopropylphenyl)quaterrylen-3,4-dicarbonsäureimid | 12 | 35 | 68,7/85 | blaugrün, kristallin | >350 |
| 17 | 67,6 | N-(p-Methoxyphenyl)quaterrylen-3,4-dicarbonsäureimid | 16 | 40 | 67,9/90 | blaugrün, mikrokristallin | >350 |
| 18 | 73,8 | N-Dodecylquaterrylen-3,4-dicarbonsäureimid | 12 | 35 | 67,0/82 | blaugrün, kristallin | >350 |

| | | | | | | | |
|---|---|---|---|---|---|---|---|
| Analytische Daten zu Beispiel 13: 11-Brom-N-(2,6-diisopropylphenyl)terrylen-3,4-dicarbonsäureimid: Elementaranalyse (Gew.-% ber./gef.): C: 77,2/77,0; H: 4,4/4,4; N: 2,05/2,1; 0: 4,7/4,7; Br: 11,7/11,8; Masse (FD, 8kV): m/z = 684,6 (M⁺, 100 %); IR (KBr): v = 1686 (s, C=O), 1645 (s, C=O) cm⁻¹; UV/VIS (NMP) : λₘₐₓ (ε) = 578 (46018), 643 (52323) nm. Analytische Daten zu Beispiel 16: 13-Brom-N-(2,6-diisopropylphenyl)quaterrylen-3,4-dicarbonsäure-imid: Elementaranalyse (Gew.-% ber./gef.): C: 80,2/80,5; H: 4,2/4,2; N: 1,7/1,7; O: 4,0/4,1; Br: 9,9/9,5; Masse (FD, 8kV) : m/z = 808,7 (M⁺, 100 %); IR (KBr): ν = 1689 (s, C=O) , 1650 (s, C=O) cm⁻¹ ; UV/VIS (NMP) : λₘₐₓ (ε) = 671 (80081), 748 (88256) nm. | | | | | | | |

## Patentansprüche

1. Rylenderivate der allgemeinen Formel I 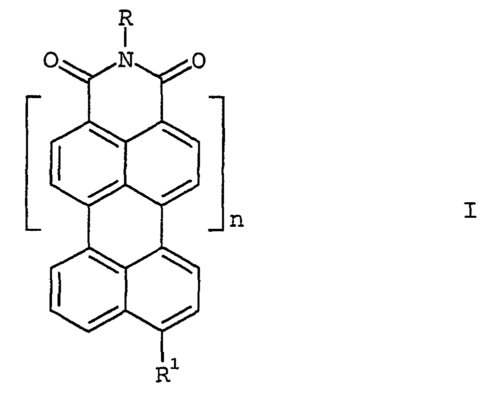 in der die Variablen folgende Bedeutung haben:
R Wasserstoff;
C₁-C₃₀-Alkyl, dessen Kohlenstoffkette durch eine oder mehrere Gruppierungen -O-, -S-, -NR²-, -CO- und/oder -SO₂- unterbrochen sein kann und das durch Carboxy, Sulfo, Hydroxy, Cyano, C₁-C₆-Alkoxy und/oder einen über ein Stickstoffatom gebundenen, 5- bis 7-gliedrigen heterocyclischen Rest, der weitere Heteroatome enthalten und aromatisch sein kann, ein- oder mehrfach substituiert sein kann;
Aryl oder Hetaryl, das durch C₁-C₁₈-Alkyl, C₁-C₆-Alkoxy, Halogen, Hydroxy, Cyano, Carboxy, -CONHR³, -NHCOR³ und/oder Aryl- oder Hetarylazo, das jeweils durch C₁-C₁₀-Alkyl, C₁-C₆-Alkoxy, Halogen, Hydroxy, Cyano und/oder Carboxy substituiert sein kann, ein- oder mehrfach substituiert sein kann;
R¹ Wasserstoff oder Brom;
R² Wasserstoff oder C₁-C₆-Alkyl;
R³ Wasserstoff; C₁-C₁₈-Alkyl; Aryl oder Hetaryl, das jeweils durch C₁-C₈-Alkyl, C₁-C₆-Alkoxy, Halogen, Hydroxy und/oder Cyano substituiert sein kann;
n 2 oder 3.

2. Rylenderivate der Formel I nach Anspruch 1, in der die Variablen die folgende Bedeutung haben:
R C₁-C₃₀-Alkyl, dessen Kohlenstoffkette durch eine oder mehrere Gruppierungen -O-, -S-, -NR²-, -CO- und/oder -SO₂- unterbrochen sein kann und das durch Hydroxy, Cyano, C₁-C₆-Alkoxy und/oder einen über ein Stickstoff-atom gebundenen, 5- bis 7-gliedrigen heterocyclischen Rest, der weitere Heteroatome enthalten und aromatisch sein kann, ein- oder mehrfach substituiert sein kann; Aryl oder Hetaryl, das durch C₁-C₁₈-Alkyl, C₁-C₆-Alkoxy, Hydroxy, Cyano, -CONHR³, und/oder -NHCOR³ ein- oder mehrfach substituiert sein kann;
R¹ Wasserstoff oder Brom;
R² Wasserstoff oder C₁-C₆-Alkyl;
R³ Wasserstoff; C₁-C₁₈-Alkyl; Aryl oder Hetaryl, das jeweils durch C₁-C₈-Alkyl, C₁-C₆-Alkoxy und/oder Cyano substituiert sein kann;
n 2 oder 3.

3. Verfahren zur Herstellung von Rylenderivaten der Formel I gemäß Anspruch 1 oder 2, **dadurch gekennzeichnet, daß** man
a) ein asymmetrisches Rylentetracarbonsäurediimid der allgemeinen Formel II 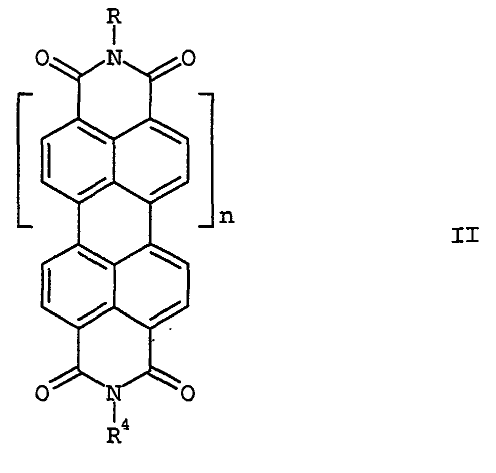 in der R⁴ C₅-C₈-Cycloalkyl, dessen Kohlenstoffgerüst durch eine oder mehrere Gruppierungen -O-, -S- und/oder -NR²- unterbrochen sein kann und das durch C₁-C₆-Alkyl ein- oder mehrfach substituiert sein kann, bedeutet,
unter alkalischen Bedingungen in Gegenwart eines polaren organischen Lösungsmittels einseitig verseift,
b) das in Schritt a) gebildete Rylentetracarbonsäuremonoimidmonoanhydrid der allgemeinen Formel III 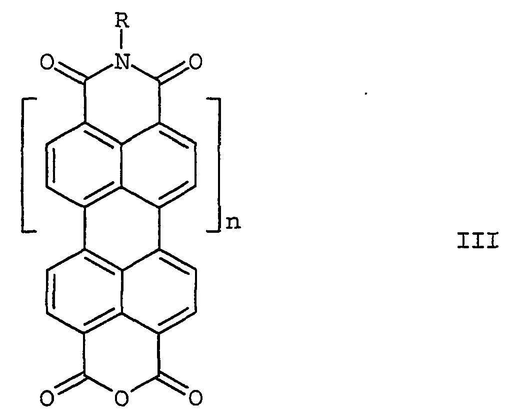 in Gegenwart einer tertiären stickstoffbasischen Verbindung und eines Übergangsmetallkatalysators einseitig decarboxyliert und
c) das Reaktionsprodukt gewünschtenfalls abschließend mit elementarem Brom umsetzt.

4. Verfahren zur Herstellung von Rylentetracarbonsäuremonoimidmonoanhydriden der allgemeinen Formel III 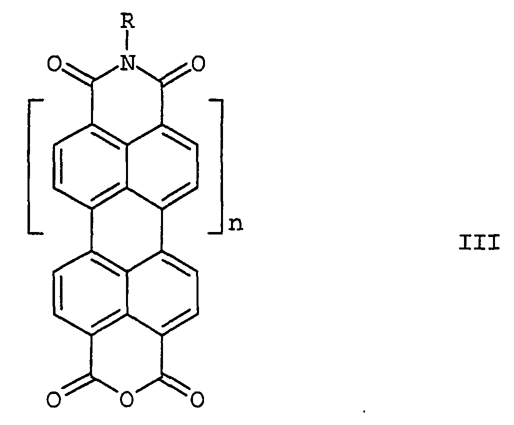 in der die Variablen folgende Bedeutung haben:
R Wasserstoff;
C₁-C₃₀-Alkyl, dessen Kohlenstoffkette durch eine oder mehrere Gruppierungen -O-, -S-, -NR²-, -CO- und/oder -SO₂- unterbrochen sein kann und das durch Carboxy, Sulfo, Hydroxy, Cyano, C₁-C₆-Alkoxy und/oder einen über ein Stickstoffatom gebundenen, 5- bis 7-gliedrigen heterocyclischen Rest, der weitere Heteroatome enthalten und aromatisch sein kann, ein- oder mehrfach substituiert sein kann;
Aryl oder Hetaryl, das durch C₁-C₁₈-Alkyl, C₁-C₆-Alkoxy, Halogen, Hydroxy, Cyano, Carboxy, -CONHR³, -NHCOR³ und/oder Aryl- oder Hetarylazo, das jeweils durch C₁-C₁₀-Alkyl, C₁-C₆-Alkoxy, Halogen, Hydroxy, Cyano und/oder Carboxy substituiert sein kann, ein- oder mehrfach substituiert sein kann;
R² Wasserstoff oder C₁-C₆-Alkyl;
R³ Wasserstoff; C₁-C₁₈-Alkyl; Aryl oder Hetaryl, das jeweils durch C₁-C₈-Alkyl, C₁-C₆-Alkoxy, Halogen, Hydroxy oder Cyano substituiert sein kann;
n 2 oder 3,
**dadurch gekennzeichnet, daß** man ein asymmetrisches Rylentetracarbonsäurediimid der allgemeinen Formel II 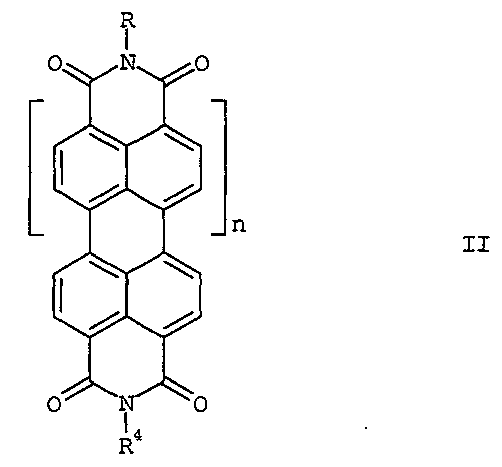 in der R⁴ C₅-C₈-Cycloalkyl, dessen Kohlenstoffgerüst durch eine oder mehrere Gruppierungen -O-, -S- und/oder -NR²- unterbrochen sein kann und das durch C₁-C₆-Alkyl ein- oder mehrfach substituiert sein kann, bedeutet,
unter alkalischen Bedingungen in Gegenwart eines polaren organischen Lösungsmittels einseitig verseift.

5. Rylentetracarbonsäuremonoimidmonoanhydride der allgemeinen Formel III 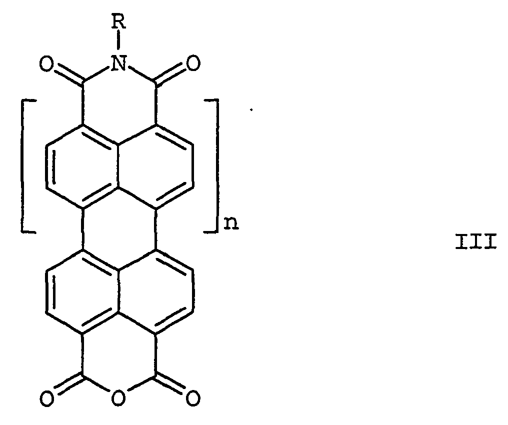 in der die Variablen folgende Bedeutung haben:
R Wasserstoff;
C₁-C₃₀-Alkyl, dessen Kohlenstoffkette durch eine oder mehrere Gruppierungen -O-, ,-S-, -NR²-, -CO- und/oder -SO₂- unterbrochen sein kann und das durch Carboxy, Sulfo, Hydroxy, Cyano, C₁-C₆-Alkoxy und/oder einen über ein Stickstoffatom gebundenen, 5- bis 7-gliedrigen heterocyclischen Rest, der weitere Heteroatome enthalten und aromatisch sein kann, ein- oder mehrfach substituiert sein kann;
Aryl oder Hetaryl, das durch C₁-C₁₈-Alkyl, C₁-C₆-Alkoxy, Halogen, Hydroxy, Cyano, Carboxy, -CONHR³, -NHCOR³ und/oder Aryl- oder Hetarylazo, das jeweils durch C₁-C₁₀-Alkyl, C₁-C₆-Alkoxy, Halogen, Hydroxy, Cyano und/oder Carboxy substituiert sein kann, ein- und/oder mehrfach substituiert sein kann;
R² Wasserstoff oder C₁-C₆-Alkyl;
R³ Wasserstoff; C₁-C₁₈-Alkyl ; Aryl oder Hetaryl, das jeweils durch C₁-C₈-Alkyl, C₁-C₆-Alkoxy, Halogen, Hydroxy und/oder Cyano substituiert sein kann;
n 2 oder 3.

6. Verwendung von Rylenderivaten der allgemeinen Formel I gemäß Anspruch 1 oder 2 zur Einfärbung von hochmolekularen organischen und anorganischen Materialien.

7. Verwendung nach Anspruch 6, **dadurch gekennzeichnet, daß** Kunststoffe, Lacke und Druckfarben und oxidische Schichtsystemen eingefärbt werden.

8. Verwendung von Rylenderivaten der allgemeinen Formel I gemäß Anspruch 1 oder 2 als Dispergierhilfsmittel, Pigmentadditive für organische Pigmente und Zwischenprodukte für die Herstellung von Fluoreszenzfarbmitteln und Pigmentadditiven.

9. Verwendung von Rylenderivaten der allgemeinen Formel I gemäß Anspruch 1 oder 2 als farbgebende Komponente in der dekorativen Kosmetik.

10. Verwendung von Rylenderivaten der allgemeinen Formel I gemäß Anspruch 1 oder 2 zur Herstellung farbiger oder im nahinfraroten Bereich des elektromagnetischen Spektrums absorbierender und/oder emittierender wäßriger Polymerisatdispersionen.

11. Verwendung von Rylenderivaten der allgemeinen Formel I gemäß Anspruch 1 oder 2 als Photoleiter in der Elektrophotographie, als Emitter in Elektro- und Chemilumineszenzanwendungen, als Aktivkomponenten in der Fluoreszenzkonversion und als Laserfarbstoffe.

12. Verwendung von Rylentetracarbonsäuremonoimidmonoanhydriden der allgemeinen Formel III gemäß Anspruch 5 zur Einfärbung von hochmolekularen organischen und anorganischen Materialien.

13. Verwendung von Rylentetracarbonsäuremonoimidmonoanhydriden der allgemeinen Formel III gemäß Anspruch 5 als Dispergierhilfsmittel, Pigmentadditive für organische Pigmente und Zwischenprodukte für die Herstellung polymerisierbarer Farbmittel und Pigmentadditive.

14. Verwendung von Rylentetracarbonsäuremonoimidmonoanhydriden der allgemeinen Formel III gemäß Anspruch 5 als farbgebende Komponente in der dekorativen Kosmetik.

15. Verwendung von Rylentetracarbonsäuremonoimidmonoanhydriden der allgemeinen Formel III gemäß Anspruch 5 zur Herstellung farbiger oder im nahinfraroten Bereich des elektromagnetischen Spektrums absorbierender und/oder emittierender wäßriger Polymerisatdispersionen.

16. Verwendung von Rylentetracarbonsäuremonoimidmonoanhydriden der allgemeinen Formel III gemäß Anspruch 5 als Photoleiter in der Elektrophotographie, als Emitter in Elektro- und Chemilumineszenzanwendungen, als Aktivkomponenten in der Fluoreszenzkonversion und als Laserfarbstoffe.

## Claims

1. A rylene derivative of the general formula I 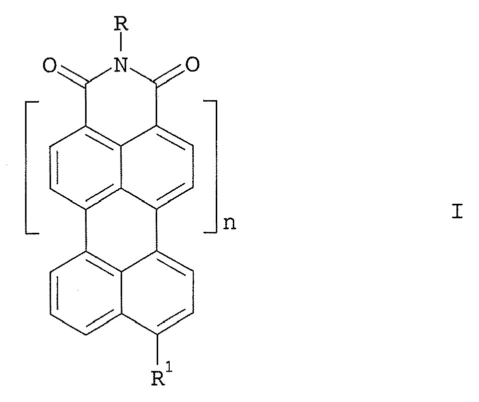 where the variables have the following meanings:
R is hydrogen;
C₁-C₃₀-alkyl, whose carbon chain may be interrupted by one or more -O-, -S-, -NR²-, -CO- and/or -SO₂- groups and which may be monosubstituted or polysubstituted by carboxyl, sulfo, hydroxyl, cyano, C₁-C₆-alkoxy and/or by a 5- to 7-membered heterocyclic radical which is bonded via a nitrogen atom, may comprise further heteroatoms and may be aromatic;
aryl or hetaryl, each of which may be monosubstituted or polysubstituted by C₁-C₁₈-alkyl, C₁-C₆-alkoxy, halogen, hydroxyl, cyano, carboxyl, -CONHR³, -NHCOR³ and/or aryl-or hetarylazo, each of which may be substituted by C₁-C₁₀-alkyl, C₁-C₆-alkoxy, halogen, hydroxyl, cyano and/or carboxyl;
R¹ is hydrogen or bromine;
R² is hydrogen or C₁-C₆-alkyl;
R³ is hydrogen; C₁-C₁₈-alkyl; aryl or hetaryl, each of which may be substituted by C₁-C₈-alkyl, C₁-C₆-alkoxy, halogen, hydroxyl and/or cyano; and
n is 2 or 3.

2. The rylene derivative of the formula I according to claim 1, where the variables have the following meanings:
R is C₁-C₃₀-alkyl, whose carbon chain may be interrupted by one or more -O-, -S-, -NR²-, -CO- and/or -SO₂- groups and which may be monosubstituted or polysubstituted by hydroxyl, cyano, C₁-C₆-alkoxy and/or by a 5- to 7-membered heterocyclic radical which is bonded via a nitrogen atom, may comprise further heteroatoms and may be aromatic;
aryl or hetaryl, each of which may be monosubstituted or polysubstituted by C₁-C₁₈-alkyl, C₁-C₆-alkoxy, hydroxyl, cyano, -CONHR³ and/or -NHCOR³;
R¹ is hydrogen or bromine;
R² is hydrogen or C₁-C₆-alkyl;
R³ is hydrogen; C₁-C₁₈-alkyl; aryl or hetaryl, each of which may be substituted by C₁-C₈-alkyl, C₁-C₆-alkoxy or cyano; and
n is 2 or 3.

3. A process for the preparation of a rylene derivative of the formula I according to claim 1 or 2, which comprises
a) monohydrolyzing an asymmetrical rylenetetracarboxylic diimide of the general formula II 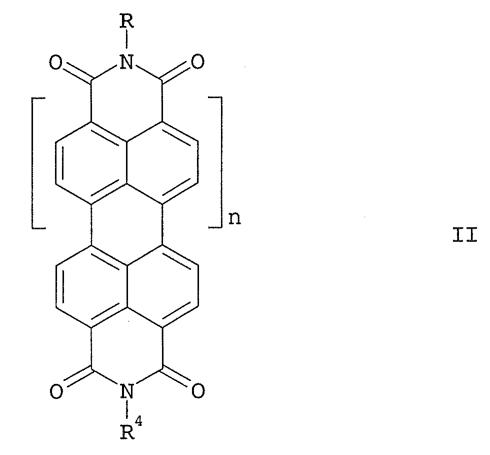 where R⁴ is C₅-C₈-cycloalkyl, whose carbon skeleton may be interrupted by one or more -O-, -S- and/or -NR²-groups and which may be monosubstituted or polysubstituted by C₁-C₆-alkyl,
under alkaline conditions in the presence of a polar organic solvent,
b) monodecarboxylating the rylenetetracarboxylic monoimide monoanhydride of the general formula III 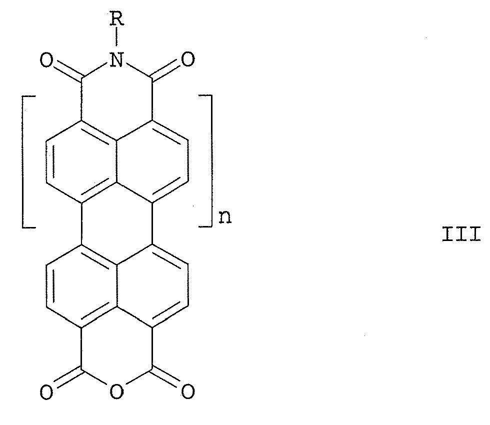 formed in step a) in the presence of a tertiary nitrogen-basic compound and in the presence of a transition-metal catalyst, and
c) if desired, subsequently reacting the reaction product with elemental bromine.

4. A process for the preparation of a rylenetetracarboxylic monoimide monoanhydride of the general formula III 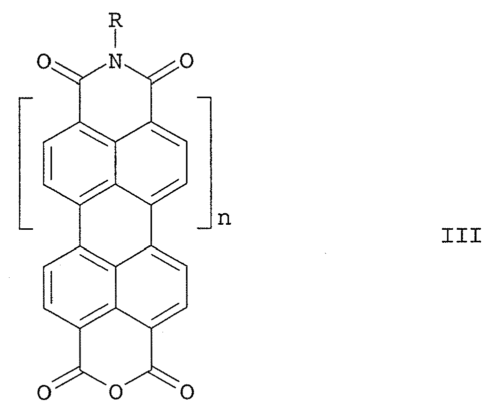 where the variables have the following meanings:
R is hydrogen;
C₁-C₃₀-alkyl, whose carbon chain may be interrupted by one or more -O-, -S-, -NR²-, -CO- and/or -SO₂- groups and which may be monosubstituted or polysubstituted by carboxyl, sulfo, hydroxyl, cyano, C₁-C₆-alkoxy and/or by a 5- to 7-membered heterocyclic radical which is bonded via a nitrogen atom, may comprise further heteroatoms and may be aromatic;
aryl or hetaryl, each of which may be monosubstituted or polysubstituted by C₁-C₁₈-alkyl, C₁-C₆-alkoxy, halogen, hydroxyl, cyano, carboxyl, -CONHR³, -NHCOR³ and/or aryl-or hetarylazo, each of which may be substituted by C₁-C₁₀-alkyl, C₁-C₆-alkoxy, halogen, hydroxyl, cyano and/or carboxyl;
R² is hydrogen or C₁-C₆-alkyl ;
R³ is hydrogen; C₁-C₁₈-alkyl; aryl or hetaryl, each of which may be substituted by C₁-C₈-alkyl, C₁-C₆-alkoxy, halogen, hydroxyl or cyano; and
n is 2 or 3,
which comprises monohydrolyzing an asymmetrical rylenetetracarboxylic diimide of the general formula II 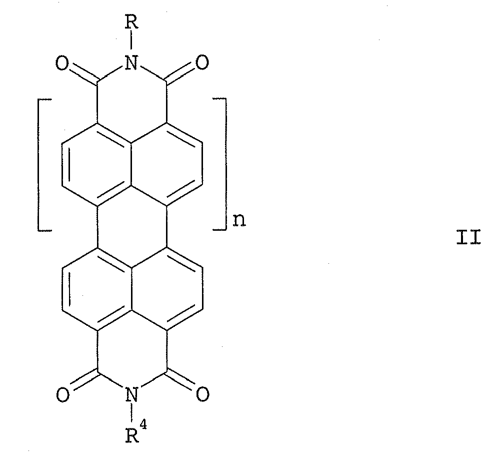 where R⁴ is C₅-C₈-cycloalkyl, whose carbon skeleton may be interrupted by one or more -O-, -S- and/or -NR²- groups and which may be monosubstituted or polysubstituted by C₁-C₆-alkyl,
under alkaline conditions in the presence of a polar organic solvent.

5. A rylenetetracarboxylic monoimide monoanhydride of the general formula III 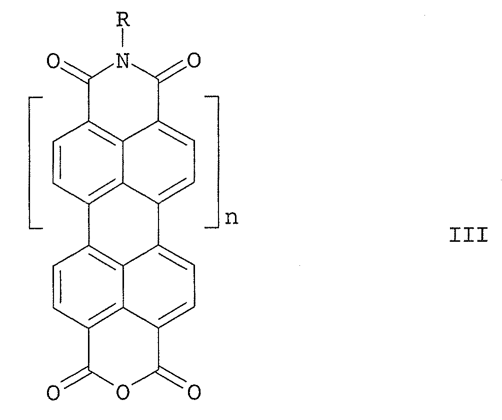 where the variables have the following meanings:
R is hydrogen;
C₁-C₃₀-alkyl, whose carbon chain may be interrupted by one or more -O-, -S-, -NR²-, -CO- and/or -SO₂- groups and which may be monosubstituted or polysubstituted by carboxyl, sulfo, hydroxyl, cyano, C₁-C₆-alkoxy and/or by a 5- to 7-membered heterocyclic radical which is bonded via a nitrogen atom, may comprise further heteroatoms and may be aromatic;
aryl or hetaryl, each of which may be monosubstituted or polysubstituted by C₁-C₁₈-alkyl, C₁-C₆-alkoxy, halogen, hydroxyl, cyano, carboxyl, -CONHR³, -NHCOR³ and/or aryl-or hetarylazo, each of which may be substituted by C₁-C₁₀-alkyl, C₁-C₆-alkoxy, halogen, hydroxyl, cyano and/or carboxyl;
R² is hydrogen or C₁-C₆-alkyl;
R³ is hydrogen; C₁-C₁₈-alkyl ; aryl or hetaryl, each of which may be substituted by C₁-C₈-alkyl, C₁-C₆-alkoxy, halogen, hydroxyl and/or cyano; and
n is 2 or 3.

6. The use of a rylene derivative of the general formula I according to claim 1 or 2 for coloring high-molecular-weight organic and inorganic materials.

7. The use according to claim 6, wherein plastics, surface coatings and printing inks and oxidic layer systems are colored.

8. The use of a rylene derivative of the general formula I according to claim 1 or 2 as dispersion aid, pigment additive for organic pigments or intermediate for the preparation of fluorescent colorants and pigment additives.

9. The use of a rylene derivative of the general formula I according to claim 1 or 2 as coloring component in decorative cosmetics.

10. The use of a rylene derivative of the general formula I according to claim 1 or 2 for the preparation of aqueous polymer dispersions which are colored or absorb and/or emit in the near infrared region of the electromagnetic spectrum.

11. The use of a rylene derivative of the general formula I according to claim 1 or 2 as photoconductor in electrophotography, as emitter in electroluminescence and chemiluminescence applications, as active component in fluorescence conversion or as laser dye.

12. The use of a rylenetetracarboxylic monoimide monoanhydride of the general formula III according to claim 5 for coloring high-molecular-weight organic and inorganic materials.

13. The use of a rylenetetracarboxylic monoimide monoanhydride of the general formula III according to claim 5 as dispersion aid, pigment additive for organic pigments or intermediate for the preparation of polymerizable colorants and pigment additives.

14. The use of a rylenetetracarboxylic monoimide monoanhydride of the general formula III according to claim 5 as coloring component in decorative cosmetics.

15. The use of a rylenetetracarboxylic monoimide monoanhydride of the general formula III according to claim 5 for the preparation of aqueous polymer dispersions which are colored or absorb and/or emit in the near infrared region of the electromagnetic spectrum.

16. The use of a rylenetetracarboxylic monoimide monoanhydride of the general formula III according to claim 5 as photoconductor in electrophotography, as emitter in electroluminescence and chemiluminescence applications, as active component in fluorescence conversion or as laser dye.

## Revendications

1. Dérivés de rylène de formule générale I 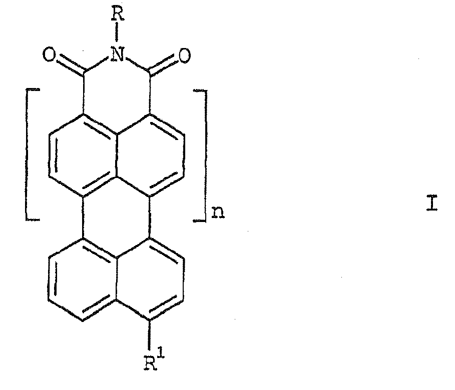 dans laquelle les variables ont la signification suivante :
R est hydrogène;
un alkyle en C₁-C₃₀ dont la chaîne carbone peut être interrompue par un ou plusieurs groupements -O-, -S-, -NR²-, -CO- et/ou -SO₂- et qui peut être une ou plusieurs fois substitué par un carboxy, un sulfo, un hydroxy, un cyano, un alcoxy en C₁-C₆ et/ou un radical hétérocyclique de 5 à 7 membres lié par un atome d'azote, qui peut contenir d'autres hétéroatomes et peut être aromatique;
un aryle ou hétaryle qui peut être une ou plusieurs fois substitué par un alkyle en C₁-C₁₈, un alcoxy en C₁-C₆, un halogène, un hydroxy, un cyano, un carboxy, -CONHR³, -NHCOR³ et/ou un aryl- ou hétarylazo, qui peut être chaque fois substitué par un alkyle en C₁-C₁₀, un alcoxy en C₁-C₆, un halogène, un hydroxy, un cyano et/ou un carboxy;
R¹ est hydrogène ou brome;
R² est hydrogène ou un alkyle en C₁-C₆;
R³ est hydrogène; un alkyle en C₁-C₁₈; un aryle ou hétaryle, qui peut être substitué chaque fois par un alkyle en C₁-C₈, un alcoxy en C₁-C₆; un halogène, un hydroxy et/ou un cyano;
n est 2 ou 3.

2. Dérivés de rylène de formule I selon la revendication 1, dans laquelle les variables ont la signification suivante :
R est un alkyle en C₁-C₃₀ dont la chaîne carbone peut être interrompue par un ou plusieurs groupements -O-, -S-, -NR²-, -CO- et/ou -SO₂ et qui peut être une ou plusieurs fois substitué par un hydroxy, un cyano, un alcoxy en C₁-C₆ et/ou un radical hétérocyclique de 5 à 7 membres lié par un atome d'azote, qui peut contenir d'autres hétéroatomes et peut être aromatique; un aryle ou hétaryle, qui peut être une ou plusieurs fois substitué par un alkyle en C₁-C₁₈, un alcoxy en C₁-C₆, un hydroxy, un cyano, -CONHR³ et/ou -NHCOR³;
R¹ est hydrogène ou brome;
R² est hydrogène ou un alkyle en C₁-C₆;
R³ est hydrogène*;* un alkyle en C₁-C₁₈*;* un aryle ou hétaryle, qui peut être substitué chaque fois par un alkyle en C₁-C₈, un alcoxy en C₁-C₆ et/ou un cyano;
n est 2 ou 3.

3. Procédé de préparation de dérivés de rylène de formule I selon la revendication 1 ou 2, **caractérisé en ce que**
a) on saponifie d'un côté seulement, dans des conditions alcalines et en présence d'un solvant organique polaire, un diimide d'acide rylène tétracarboxylique asymétrique de formule générale II 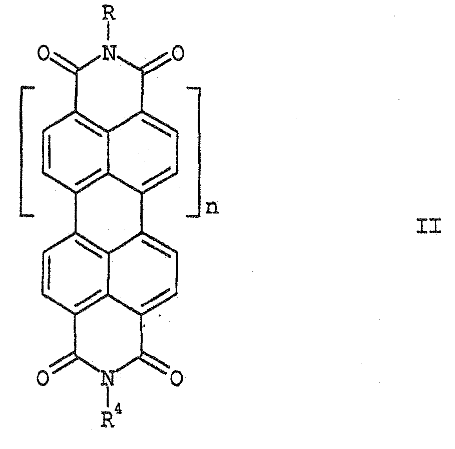 dans laquelle R⁴ signifie un cycloalkyle en C₅-C₈ dont la structure carbone peut être interrompue par un ou plusieurs groupements -O-, -S- et/ou -NR²- et qui peut être une ou plusieurs fois substitué par un alkyle en C₁-C₆,
b) on décarboxylise d'un côté seulement en présence d'un composé tertiaire basique azote et d'un catalyseur de métal de transition le monoimidomonoanhydride d'acide rylène tétracarboxylique formé à l'étape a) et de formule générale III 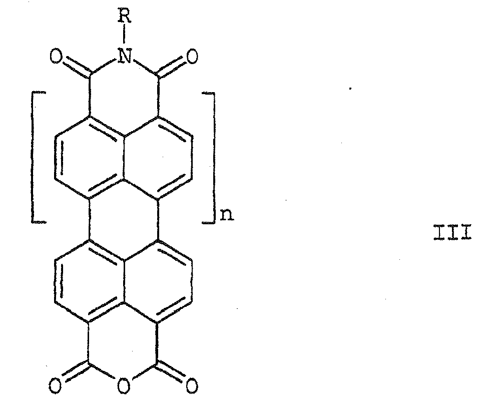 et
c) on fait réagir pour terminer et si on le souhaite le produit réactionnel avec du brome élémentaire.

4. Procédé de préparation de monoimidomonoanhydrides d'acide rylène tétracarboxylique de formule générale III 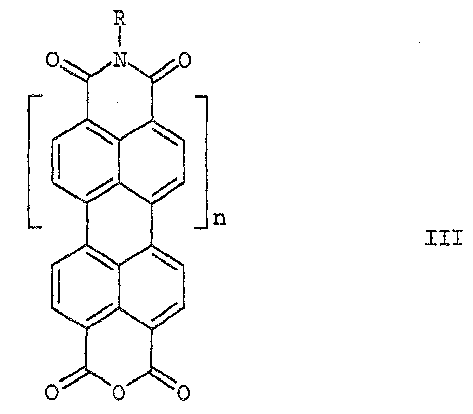 dans laquelle les variables ont la signification suivante :
R est hydrogène;
un alkyle en C₁-C₃₀ dont la chaîne carbone peut être interrompue par un ou plusieurs groupements -O-, -S-, -NR²-, -CO- et/ou -SO₂- et qui peut être une ou plusieurs fois substitué par un carboxy, un sulfo, un hydroxy, un cyano, un alcoxy en C₁-C₆ et/ou un radical hétérocyclique de 5 à 7 membres lié par un atome d'azote, qui peut contenir d'autres hétéroatomes et peut être aromatique;
un aryle ou hétaryle qui peut être une ou plusieurs fois substitué par un alkyle en C₁-C₁₈, un alcoxy en C₁-C₆, un halogène, un hydroxy, un cyano, un carboxy, -CONHR³, -NHCOR³ et/ou un aryl- ou hétarylazo, qui peut être chaque fois substitué par un alkyle en C₁-C₁₀, un alcoxy en C₁-C₆, un halogène, un hydroxy, un cyano et/ou un carboxy;
R² est hydrogène ou un alkyle en C₁-C₆;
R³ est hydrogène; un alkyle en C₁-C₁₈; un aryle ou hétaryle, qui peut être substitué chaque fois par un alkyle en C₁-C₈, un alcoxy en C₁-C₆, un halogène, un hydroxy ou un cyano;
n est 2 ou 3.
**caractérisé en ce qu'**on saponifie d'un côté seulement dans des conditions alcalines et en présence d'un solvant organique polaire un diimide d'acide rylène tétracarboxylique de formule générale II 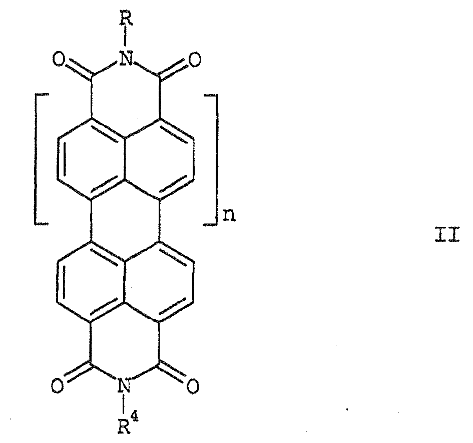 dans laquelle R⁴ signifie un cycloalkyle en C₅-C₈ dont la structure carbone peut être interrompue par un ou plusieurs groupements -O-, -S- et/ou -NR²- et qui peut être une ou plusieurs fois substitué par un alkyle en C₁-C₆.

5. Monoimidomonoanhydrides d'acide rylène tétracarboxylique de formule générale III 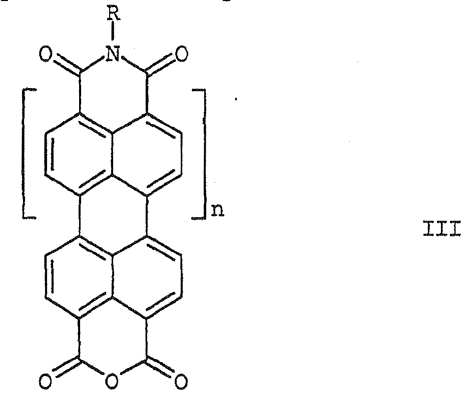 dans laquelle les variables ont la signification suivante :
R est hydrogène;
un alkyle en C₁-C₃₀ dont la chaîne carbone peut être interrompue par un ou plusieurs groupements -O-, -S-, -NR²-, -CO- et/ou -SO₂- et qui peut être une ou plusieurs fois substitué par un carboxy, un sulfo, un hydroxy, un cyano, un alcoxy en C₁-C₆ et/ou un radical hétérocyclique de 5 à 7 membres lié par un atome d'azote, qui peut contenir d'autres hétéroatomes et peut être aromatique;
un aryle ou hétaryle qui peut être une ou plusieurs fois substitué par un alkyle en C₁-C₁₈, un alcoxy en C₁-C₆, un halogène, un hydroxy, un cyano, un carboxy, -CONHR³, -NHCOR³ et/ou un aryl- ou hétarylazo, qui peut être chaque fois substitué par un alkyle en C₁-C₁₀, un alcoxy en C₁-C₆, un halogène, un hydroxy, un cyano et/ou un carboxy;
R² est hydrogène ou un alkyle en C₁-C₆ ;
R³ est hydrogène; un alkyle en C₁-C₁₈ ; un aryle ou hétaryle, qui peut être substitué chaque fois par un alkyle en C₁-C₈, un alcoxy en C₁-C₆, un halogène, un hydroxy et/ou un cyano;
n est 2 ou 3.

6. Utilisation de dérivés de rylène de formule générale I selon la revendication 1 ou 2, pour la coloration de matières organiques et inorganiques à poids moléculaire élevé.

7. Utilisation selon la revendication 6, **caractérisée en ce qu'**on colore des matières synthétiques, des peintures et des encres d'impression ainsi que des systèmes en couches oxydiques.

8. Utilisation de dérivés de rylène de formule générale I selon la revendication 1 ou 2 comme adjuvants de dispersion, additifs de pigments pour pigments organiques et produits intermédiaires pour la fabrication de colorants fluorescents et additifs de pigments.

9. Utilisation de dérivés de rylène de formule générale I selon la revendication 1 ou 2 comme composant colorant dans la cosmétique décorative.

10. Utilisation de dérivés de rylène de formule générale I selon la revendication 1 ou 2 pour la préparation de dispersions de polymères aqueuses colorées ou absorbant et/ou émettant dans le domaine de l'infrarouge proche du spectre électromagnétique.

11. Utilisation de dérivés de rylène de formule générale I selon la revendication 1 ou 2 comme photoconducteurs dans l'électrophotographie, comme émetteurs dans les applications d'électroluminescence et de chimioluminescence, comme composants actifs dans la conversion de fluorescence et comme colorants laser.

12. Utilisation de monoimidomonoanhydrides d'acide rylène tétracarboxylique de formule générale III selon la revendication 5, pour la coloration de matières organiques et inorganiques à poids moléculaire élevé.

13. Utilisation de monoimidomonoanhydrides d'acide rylène tétracarboxylique de formule générale III selon la revendication 5 comme adjuvants de dispersion, additifs de pigments pour pigments organiques et produits intermédiaires pour la fabrication de colorants polymérisables et additifs de pigments.

14. Utilisation de monoimidomonoanhydrides d'acide rylène tétracarboxylique de formule générale III selon la revendication 5 comme composant colorant dans la cosmétique décorative.

15. Utilisation de monoimidomonoanhydrides d'acide rylène tétracarboxylique de formule générale III selon la revendication 5 pour la préparation de dispersions de polymères aqueuses colorées ou absorbant et/ou émettant dans le domaine de l'infrarouge proche du spectre électromagnétique.

16. Utilisation de monoimidomonoanhydrides d'acide rylène tétracarboxylique de formule générale III selon la revendication 5 comme photoconducteurs dans l'électrophotographie, comme émetteurs dans les applications d'électroluminescence et de chimioluminescence, comme composants actifs dans la conversion de fluorescence et comme colorants laser.
